# EUROPÄISCHE PATENTSCHRIFT

(11) **EP 0 800 836 B1**
(45) Veröffentlichungstag und Bekanntmachung des Hinweises auf die Patenterteilung: **26.02.2003**
(21) Anmeldenummer: 96105847.6
(22) Anmeldetag: 14.04.1996
(51) Int. Cl.: A61M 1/06

(54) **Einrichtung zum Absaugen von Muttermilch**
Breast pump
Tire-lait

(43) Veröffentlichungstag der Anmeldung: 15.10.1997
(73) Patentinhaber: Medela AG, 6340 Baar (CH)
(72) Erfinder: Larsson, Karl-Olof Axel Helmer, 6300 Zug (CH)
(74) Vertreter: Troesch Scheidegger Werner AG

(56) Entgegenhaltungen:
- EP-A- 0 123 269
- EP-A- 0 162 358
- EP-A- 0 700 689
- GB-A- 271 857
- US-A- 4 572 104

## Beschreibung

Die vorliegende Erfindung betrifft eine Einrichtung zum Absaugen von Muttermilch mit einem Saugaggregat, zwei mit Brustaufsatzhauben und selbsttätig arbeitenden Ventilen ausgerüsteten Milch-Aufnahmebehältern und zwei vom Saugaggregat zu den Aufnahmebehältern führenden Saugleitungen.

Bei bekannten Einrichtungen dieser Art weist das Saugaggregat eine Unterdruckkammer auf, mit welcher die beiden Saugleitungen in Verbindung stehen, um ein gleichzeitiges Absaugen von Milch aus beiden Brüsten zu ermöglichen, d.h. mit der Absaugeinrichtung kann ein "paralleles" Absaugen erfolgen.

Die Absaugaggregate sind üblicherweise einfach wirkende Zylinder, welche während des Saughubes das Absaugen der Milch über die beiden Saugleitungen erlauben.

Die Milch-Aufnahmebehälter weisen eine an sich bekannte Ventilvorrichtung auf, welche bei Unterdruck in der Saugleitung automatisch schliesst und ein Absaugen erlaubt, während beim Druckhub des Kolbens des Saugaggregates die Ventile öffnen und der erzeugte Druck abgeblasen wird (z.B. durch Ventile am Zylinder).

Es hat sich nun herausgestellt, dass ein alternierendes Absaugen an den beiden Brüsten wesentliche Vorteile bringt. Damit durch den Leerhub (Druckhub) keine Zeit verloren geht, musste eine konstruktive Lösung gefunden werden, welche diesem Problem Rechnung trägt.

Aufgabe der vorliegenden Erfindung war somit, eine Einrichtung zum Absaugen von Muttermilch der eingangs definierten Art zu schaffen, welche ein alternierendes Absaugen von Milch durch zwei separate Saugleitungen ermöglicht.

Diese Aufgabe wurde bei einer Einrichtung der oben beschriebenen Art erfindungsgemäss durch die Merkmale gemäss kennzeichnendem Teil von Anspruch 1 gelöst.

Besondere Ausführungsformen der erfindungsgemässen Einrichtung sind in den abhängigen Ansprüchen definiert.

Dank dem erfindungsgemässen Konzept der Einrichtung ist es nun möglich, Milch alternierend und ohne Zeitverlust aus den zwei Brüsten abzusaugen, was für die stillende Mutter eine besonders angenehme Wirkung mit sich bringt. Der Verlauf der Saugwirkung (Unterdruck) ist dabei durchaus vergleichbar mit dem herkömmlichen Gerät, welches mit einer (oder zwei parallelen) Saugleitung(en) arbeitet, abhängig von der Geometrie des Saugaggregates und der Arbeitsgeschwindigkeit.

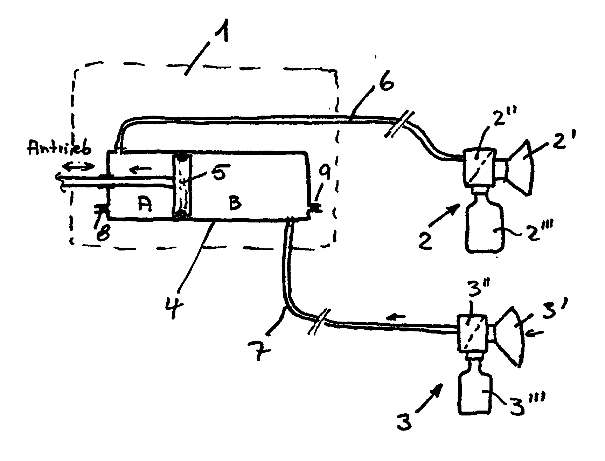

Die Erfindung wird nachstehend anhand eines in der Zeichnung dargestellten Ausführungsbeispiels noch etwas näher erläutert.

Die einzige Figur zeigt, nicht massstäblich, eine Milchabsaugeinrichtung nach der Erfindung in rein schematischer Darstellung. Die Einrichtung umfasst dabei ein Saugaggregat 1 mit einem doppelt wirkenden Arbeitszylinder 4 mit Arbeitskolben 5, welcher in den beiden Kammern A bzw. B abwechselnd einen Unterdruck erzeugen kann. Der Kolben 5 wird mittels eines an sich bekannten Antriebes (nicht dargestellt, z.B. Kurbeltrieb) motorisch oder von Hand hin- und herbewegt und erzeugt dabei abwechselnd in den Kammern A bzw. B den erforderlichen Unterdruck.

Aus jeder der Kammern A, B führt eine Saugleitung 6 bzw. 7 zu je einem Milch-Aufnahmebehälter 2 bzw. 3, deren Köpfe 2" bzw. 3" mit einer bei solchen Geräten an sich bekannten Ventileinrichtung und mit Brustaufsatzhauben 2' bzw. 3' versehen sind.

Sobald in der Saugleitung 6 bzw. 7 Druck statt Unterdruck entsteht, wird in den Köpfen 2", 3" das Ventil geöffnet (und damit die angeschlossene Brust entlastet). Der Ueberdruck in der zugehörigen Kammer A oder B entweicht z.B. über ein Rückschlagventil 8 bzw. 9.

Bei Unterdruck, d.h. Saughub des Kolbens 5, sind die Ueberdruckventile 8 bzw. 9 geschlossen wie auch die Ventile in den Köpfen 2" bzw. 3", so dass die Saugwirkung in den Brustaufsatzhauben entsteht, Milch abgesaugt wird und in den eigentlichen Aufnahmebehälter 2"' bzw. 3"' gelangt.

Das Ganze ist derart konzipiert, dass in den Saugleitungen alternierend, d.h. nie gleichzeitig, Unterdruck erzeugt wird, was logischerweise zu einem alternierenden Absaugen von Milch aus den beiden Brüsten führt.

Bei der Ausführung mit doppelt wirkendem Arbeitszylinder erfolgt bei jedem Hub eine Saugwirkung in einer der beiden Saugleitungen, so dass keine eigentlichen Arbeitspausen entstehen, die Brüste jedoch nur alternierend abgesaugt werden.

Selbstverständlich könnte anstelle des konstruktiv unaufwendigen doppelt wirkenden Arbeitszylinders auch ein Absaugaggregat mit zwei voneinander unabhängigen Saugkammern verwendet werden, welches in den beiden Kammern abwechselnd Unterdruck erzeugt.

Dank der erfindungsgemässen Einrichtung wird eine völlig neue Art des Absaugens von Muttermilch ermöglicht, was besonders für die stillende Mutter grosse Vorteile und Annehmlichkeiten mit sich bringt.

## Patentansprüche

1. Einrichtung zum Absaugen von Muttermilch mit einem Saugaggregat (1), zwei mit Brustaufsatzhauben (2', 3') und selbsttätig arbeitenden Ventilen ausgerüsteten Milch-Aufnahmebehältern (2; 3) und zwei vom Saugaggregat (1) zu den Aufnahmebehältern (2; 3) führenden Saugleitungen (6; 7), **dadurch gekennzeichnet, dass** das Saugaggregat (1) zwei Kammern (A; B) aufweist, in welchen abwechselnd ein Unterdruck erzeugt wird, und an welche je eine der beiden Saugleitungen (6; 7) angeschlossen ist, so dass in jeder Saugleitung (6; 7) alternierend ein Unterdruck erzeugt wird, welcher das Absaugen von Muttermilch bewirkt.

2. Einrichtung nach Anspruch 1, **dadurch gekennzeichnet, dass** das Saugaggregat (1) einen doppelt wirkenden Arbeitszylinder (4) umfasst, dessen beide Arbeitskammern (A; B) an die Saugleitungen (6; 7) angeschlossen sind.

3. Einrichtung nach Anspruch 2, **dadurch gekennzeichnet, dass** der Kolben (5) des Arbeitszylinders (4) von Hand oder motorisch antreibbar ist.

## Claims

1. Device for extracting breast milk, having a suction unit (1), two breast attachment caps (2', 3') and milk-receiving containers (2; 3) equipped with automatically operating valves, and two suction lines (6; 7) leading from the suction unit (1) to the receiving containers (2; 3), **characterized in that** the suction unit (1) has two chambers (A; B), in which a negative pressure is generated in an alternating manner and to which one of the two suctions lines (6; 7) is connected in each case, so that a negative pressure, which brings about the extraction of breast milk, is generated in an alternating manner in each suction line (6; 7).

2. Device according to Claim 1, **characterized in that** the suction unit (1) comprises a double action working cylinder (4), the two working chambers (A; B) of which are connected to the suction lines (6; 7).

3. Device according to Claim 2, **characterized in that** the piston (5) of the working cylinder (4) can be driven manually or by motor.

## Revendications

1. Dispositif d'aspiration de lait maternel avec un appareil d'aspiration (1), deux récipients de collecte de lait munis de capots à placer sur le sein (2',3') et de vannes fonctionnant automatiquement et deux conduits d'aspiration (6,7) menant de l'appareil d'aspiration (1) aux récipients de collecte (2;3), **caractérisé en ce que** l'appareil d'aspiration (1) présente deux chambres (A; B) dans lesquelles est produit alternativement une dépression et auxquelles est raccordé respectivement l'un des deux conduits d'aspiration (6;7) de telle sorte que dans chaque conduit d'aspiration (6;7), en alternance une dépression est produite qui provoque l'aspiration du lait maternel.

2. Dispositif selon la revendication 1, **caractérisé en ce que** l'appareil d'aspiration (1) comprend un vérin de travail (4) à action double dont les deux chambres de travail (A; B) sont raccordées aux conduits d'aspiration (6; 7).

3. Installation selon la revendication 2, **caractérisée en ce que** le piston (5) du vérin de travail (4) peut être entraîné à la main ou par moteur.
